# EUROPEAN PATENT APPLICATION

(11) **EP 3 329 954 A1**
(43) Date of publication of application: **06.06.2018**
(21) Application number: 17203498.5
(22) Date of filing: 24.11.2017
(51) Int. Cl.: A61M 5/42, A61B 17/00

(54) **AN ANTI-PAIN DEVICE FOR INJECTIONS WITH SECURITY SYSTEM**

(30) Priority: 24.11.2016 IT 201600119249
(71) Applicant: Rosti, Luca Dario Marco, 20122 Milano (IT)
(72) Inventor: Rosti, Luca Dario Marco, 20122 Milano (IT)
(74) Representative: Croce, Valeria

(57) **Abstract**

The object of the present invention is an anti-pain device (10) for injections and/or sampling in a patient comprising a flexible plate-shaped element (1) with an upper face (2) and a lower face (3) which join in a peripheral edge (4), said lower face (3) comprising a plurality of skin stimulating elements (6) and said upper face (2) comprising gripping, protecting and pressing elements, said device being characterised in that it does not comprise: automatic means capable of producing vibrations or images or sounds and being further characterised in that it does not comprise elements or means for puncturing the skin of a patient and/or elements or means for administering drugs to the patient.

## Description

The object of the present invention is an anti-pain device which finds application in the medical field, if it is necessary to carry out injections or sampling, which reduces as much as possible the pain associated with the puncture of the needle and which comprises a system for the safety of the operator.

It is known that the puncture of a needle during an injection may represent a real pain for many patients.

This is particularly true in the case of children, wherein the anxiety and the worry often involve the parent which accompanies the little patient.

For different reasons, which comprise the need to quickly carry out injections, the opportunity to prevent the child from getting frightened and starting to cry, it is essential to adopt all of the measures and the cautions which may simplify the operation.

In addition to that, the safety of the physician that carries out the injection should not be forgotten, who should avoid in any way to be punctured by the needle of the syringe.

For such purposes there are already known devices for attenuating the pain caused by the puncture of a needle.

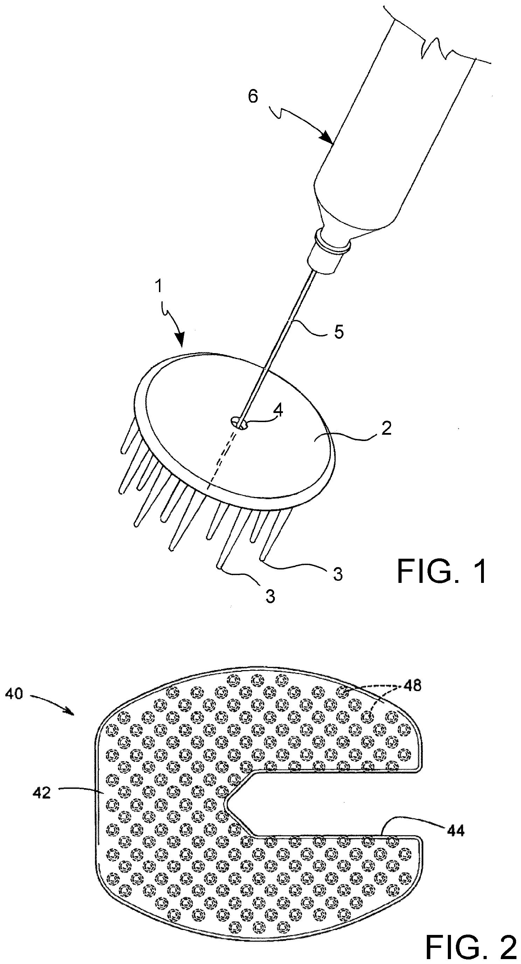

For example, the Italian patent 1.348.899 (Romanò Carlo Luca, Milan, Italy) describes the device in figure 1, which comprises a flat circular portion from which pointed elements emerge, all mutually equal, intended to be pushed on the skin at the injection point. Such a flat portion may comprise a through hole which the needle of the syringe passes, which in this way is guided. The device is said to be able to comprise a grip, for easier use of the device. However, in this regard, no further detail is provided.

Figure 2 represents the device described in document US 6.902.544 by the company Bionix Development Corporation (Toledo, OH, USA). Such a device uses the same principle of the device by Romanò Carlo Luca and is said to be able to comprise finger grips to facilitate the grip. It should be noted that in this regard neither an example nor description element have been provided. In the same way, even giving the possibility that the device comprises protection elements for the operator, no useful indication is provided in this respect.

The device shown in figure 3 is the object of the Chinese patent document CN 204275182 (Tianjin Juncheng Medical Devices, China) and substantially has the features of the device by Bionix Development Corporation, to which however it does not seem to bring about any modification nor improvement.

The US document US 3.620.209 (Harvey Kravitz, Skokie, Ill. US) describes the device shown in figure 4, which may be attached to the arm of the patient and activated by an automatic mechanism which produces a vibration capable of completely attenuating or masking the pain caused by the puncture of the syringe. Such a device has, in particular, important drawbacks, such as, for example, the need to operate with battery, the possibility of being applied to a site to which it may be attached by a bracelet, the longer time required for closing of the bracelet itself, which could even cause the patient, especially if a baby, to get nervous.

The prior art document US 4.586.924 describes a device which comprises a stiff plate element with a flat lower face. Such a configuration makes it necessary to obtain the constricting and immobilising purposes of the vein in a limb of a patient, so as to facilitate the insertion of the needed of a syringe.

The prior art document WO 2008/086552 describes a complex automatic apparatus for the injection of an intravenous administrable medium also without the assistance of specialised medical personnel. The administering medium is contained in a reservoir which is part of the same device. Even the puncturing element is comprised in the same apparatus. The injection point is necessarily within the stimulation area of the skin.

The technical problem at the basis of the present invention is therefore that of providing the skilled in the field, thus nurses and doctors, with a simple, light and manageable device, for the subcutaneous or intramuscular administration of drugs or medical preparation, in particular for administering vaccines, or for carrying out intravenous sampling/injections, which attenuate the pain caused by the puncture of the needle of a syringe, which is of easy, practical and quick use, which may be firmly gripped and which may be used on any point of the skin, with clear advantages for the operator and for the patient.

Such a problem is solved by a device for the sensory stimulation of the skin defined in the main attached claim and, in its particular aspects, in the dependent claims, the definitions thereof form an integrating part of the present description.

Further features and advantages of the device of the present invention will appear from the following description of a preferred embodiment, provided by a non-limiting example, with reference to the accompanying figures, wherein:

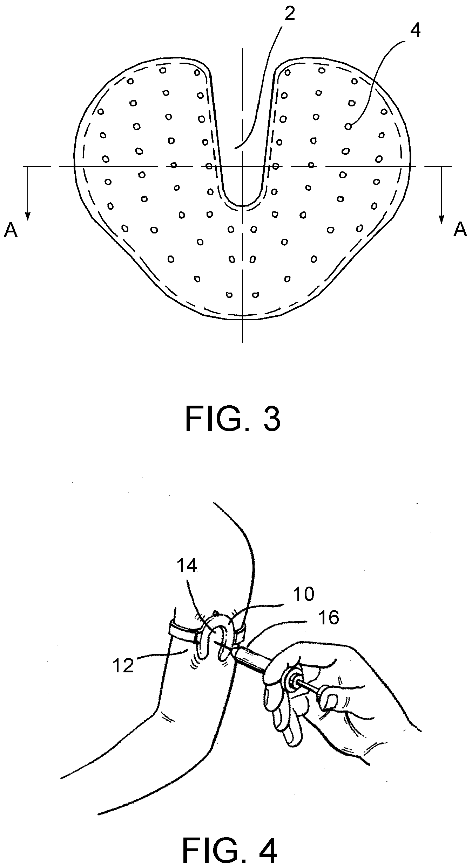

figures 1 to 4 show prior art devices, as described above;
figure 5 shows an embodiment of the device of the present invention (A=top view, B= section along line A-A, C= section along line B-B);
figure 6 shows an alternative embodiment of the device of the present invention (A=top view, B= detail of figure 6A, C= section along line A-A, D= section along line B-B).

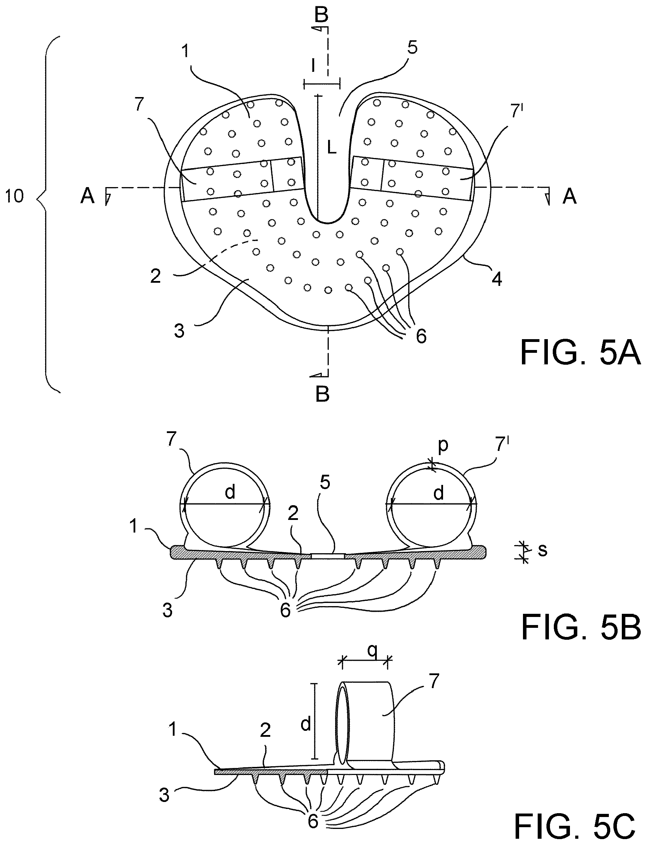

With reference to figure 5, a device according to the present invention is wholly indicated with reference numeral 10.

The device 10 is made of a plate-shaped element 1 (or plate) having a gripping or holding upper (outer) face 2 and a lower (inner) face 3 of contact with the skin of a subject, for example a patient, which join in a peripheral edge 4.

The plate of the element 10 has a generally rounded or heart-shaped or "U"-shaped shape, wherein other shapes are in any case equally possible.

According to a preferred embodiment, the peripheral edge 4 of the plate-shaped element comprises a concave portion 5 which delimits an injection area.

Such a concave portion 5 may have an elongated shape and is characterised by a length L (or depth) and by a width 1.

For the purposes of the present invention, the plate 1 is flexible, being preferably made of an elastic material such as a polymeric material.

As regards the thickness of the plate s, this is variable according to the material with which it is made of; few millimetres (2-4 mm) are generally sufficient to allow a good deformability.

The lower (or inner) face 3 of the plate 1 comprises a plurality of elements 6 for the stimulation of the skin of the patient, in the form of cylindrical, conical, frustoconical, pyramidal protrusions, or of another form ending with a slightly pointed end.

Such stimulating elements are clearly elements which stimulate the skin without penetrating it and without causing pain.

The upper (or outer) surface 2 of the plate, on the other hand, comprises gripping elements.

Typically, the grip of the device is held with the forefinger and with the middle finger of the operator hand.

In an aspect of the invention, such gripping elements are represented by roughness (not shown in the figures) which allows a firm grip of the device 10 by the operator.

According to a preferred aspect, such gripping elements further comprise gripping elements which are also protection and pressure elements.

As shown in figure 5, such elements are preferably rings 7,7', which in a first embodiment are closed while, in an alternative embodiment (not shown in the figures), are open.

The rings 7,7' are in particular characterised by an inner diameter d, by a width q and by a thickness p.

In general, the dimensions are such as to allow an easy and firm grip of the device.

To this end, a diameter d of about 1.3-2 cm (inner diameter) may be sufficient, as well as a width q from few millimetres (1-5 mm) to 1 cm or more.

In an embodiment, the gripping elements, for example represented by two open or closed rings 7,7', are made of the same material as the plate 1, optionally forming therewith a single body; alternatively, they may be made in a different material, for example less flexible and more resistant.

As for the material of the plate 1, the thickness p of the rings essentially depends on the material with which they are made of, so that the function thereof of the gripping and protection elements may be satisfied.

In a preferred aspect of the invention, the device 10 comprises two closed or open rings.

For a higher efficacy, the gripping and/or protection elements are preferably at the sides of the concavity 5 and, therefore, of the injection area.

In this way, therefore, the operator may exercise the skin stimulating action right in the points closest to the injection site.

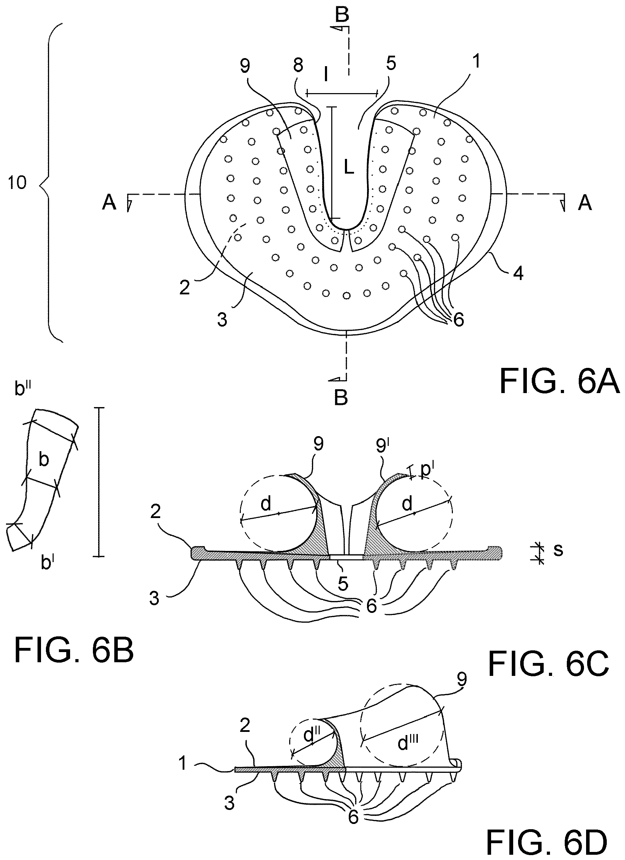

Figure 6 shows a second embodiment of the device of the present invention.

More in particular, according to such an aspect the device 10 comprises gripping elements which are also protection and pressure elements along the portion of the edge of the plate 1 which define the concavity 5 and which are in the form of walls 9 curved to form a sort of collar.

Such walls 9 are characterised in particular by a length a, by a width b, by a thickness p' and by a curvature d' (such a curvature being defined as the virtual complete diameter of a section of the wall 9,9' in a point).

As concerns the thickness p', reference shall be made to the above description for the thickness p of the rings.

In a first embodiment of the device 10 of the invention, the curve walls 9,9' are characterised by a curvature (or diameter d') constant for the entire length a, while in an alternative embodiment, the curvature is not constant (see figure 6).

According to a preferred embodiment, in fact, the curvature d" in the portion proximal to the operator may be smaller with respect to the curvature d"' of the distal portion.

With reference to the width b of each wall 9,9', this may be constant in the entire length a or b' of the proximal portion may be narrower than the width b" of the distal portion.

Advantageously, the walls 9,9' define two protecting shields for the fingers of the operator.

In a preferred aspect, such curved walls 9,9' have a length a substantially equal to the depth or length L of the concavity 5.

It is not further excluded the possibility that in the portion corresponding to about half of the length a, the wall 9,9' has an amplitude d' which narrows (that is a diameter which decreases), for the purpose of further improving the ergonomics.

In a preferred embodiment of the invention, the two curved walls 9,9' are mutually specular, having equal length a, width b and curvature d'.

However, the present invention also provides for the case wherein the two curved walls have one or more different lengths a, widths b or curvatures d'.

In a preferred embodiment, for example, a different curvature may be provided to better adapt to a right-handed or left-handed subject.

In both the embodiments above described, it may be provided that on the lower face of the plate 3 the skin stimulating elements are arranged with a nonhomogeneous density.

For example, it may be provided that such elements 6 are denser in some portions of the inner face 3 of the plate with respect to others, so as to exercise a more efficient or more precise stimulator effect.

Furthermore, according to particular aspects of the invention the device:
- comprises portions of the lower surface of the plate 3 having a different density of stimulating elements in different areas;
- comprises stimulating elements 6 of mutually different shape;
- comprises stimulating elements 6 of mutually different width;
- comprises stimulating elements 6 of mutually different length;
- comprises a concavity at least 2-3 cm long, so as to allow the formation of a bend of the skin sufficiently long for an easy and quick injection;
- comprises a concavity at least 1-1.5 cm wide, so as to allow the formation of a bend of the skin sufficiently wide to avoid causing pain to the patient;
- comprises a plate-shaped element 1 which, alone or in combination with the gripping elements 9,9', gives rise to a fantasy shape; in this way, the patient may be distracted and not realize the injection which is being carried out.

According to such embodiments, the pain relieving effect is surprisingly enhanced.

For the purposes of the present invention, it should be noted that the described device does not comprise automatic means (for example, battery-operated) capable of producing vibrations or images or sounds.

For the purposes of the present invention, it should be noted that the described device does not comprise elements or means for puncturing the skin of the patient, for example the needle of a syringe or a device for administering drugs or medical preparations, and/or elements or means for administering such drugs or medical preparations to the patient and/or reservoirs for drug or medical preparation.

In this way, the steps of administering/sampling and of attenuating pain are mutually independent, that is they may be performed by mutually different people and on mutually not matching areas; for example, the skin stimulation to obtain a pain relieving effect may be carried out in an area of the body different than that wherein the puncture takes place.

According to the present invention, in fact, the puncture site is outside the skin stimulation area.

From the description provided above, the man skilled in the art may appreciate the numerous advantages offered by the anti-pain device of the invention.

In particular, even being of simple design, the described device comprises elements which make it particularly useful and advantageous with respect to already known similar devices.

The rings and the curved walls, for example, simultaneously perform a role:
- of gripping the device by the operator, allowing to carry out the injection with greater serenity and speed;
- of protecting the operator from any punctures, also contributing in this way to the personal safety thereof;
- of pressure, therefore facilitating the patient skin stimulating action.

Of the embodiment which comprises the gripping, protection and pressure elements represented by curved walls, there may be appreciated the ease and the speed with which the operator may quickly release the fingers freeing them from the device.

The present invention is applied to the subcutaneous or intramuscular administration of drugs and medical preparations to a patient, this meaning preferably pharmaceutical products, including vaccines or also to carrying intravenous sampling/administration of pharmaceutical products.

A man skilled in the art may make several adaptations, changes and replacements of elements with other functionally equivalent to the embodiment described above of the device of the invention in order to meet incidental and specific needs, without departing from the scope of the following claims.

For example, the materials used may be plastic materials approved and authorised for the specific use.

In the same way, colours and shapes may vary according to particular needs or preferences without departing from the main function previously described.

## Claims

1. An anti-pain device (10) for injections and/or sampling in a patient comprising a flexible plate-shaped element (1) with an upper face (2) and a lower face (3) which join in a peripheral edge (4), said lower face (3) comprising a plurality of skin stimulating elements (6) and said upper face (2) comprising gripping, protecting and pressing elements, said device being **characterised in that** it does not comprise: automatic means capable of producing vibrations or images or sounds and being further **characterised in that** it does not comprise elements or means for puncturing the skin of a patient and/or elements or means for administering drugs to the patient.

2. The anti-pain device (10) according to the preceding claim wherein said peripheral edge (4) comprises a concave portion (5) which delimits an injection area on the skin of the patient.

3. The anti-pain device (10) according to claim 1 or 2, wherein said plate-shaped element (1) is made of flexible material.

4. The anti-pain device (10) according to any one of the preceding claims, wherein said skin stimulating elements (6) comprise protrusions having a cylindrical, conical, frustoconical, pyramidal shape or having another shape ending with a pointed end.

5. The anti-pain device (10) according to any one of the preceding claims, wherein said lower face (3) comprises said skin stimulating elements (6) arranged with different density on said lower face (3) and/or with a mutually different shape and/or with a mutually different width and/or with a mutually different length.

6. The anti-pain device (10) according to any one of the preceding claims, wherein said gripping, protecting and pressing elements are located at the sides of the concave portion (5).

7. The anti-pain device (10) according to any one of the preceding claims, wherein said gripping, protecting and pressing elements are in the form of open or closed rings (7,7').

8. The anti-pain device (10) according to any one of the preceding claims, wherein said gripping, protecting and pressing elements are in the form of curved walls (9,9') having a length (a), a width (b) and a diameter (d).

9. The anti-pain device (10) according to the preceding claim, wherein said gripping, protecting and pressing elements in the form of curved walls (9,9') having a width (b) and/or a diameter (d) constant or non-constant for the entire length (a).

10. The anti-pain device (10) according to any one of the preceding claims, wherein said plate-shaped element (1), optionally in combination with said gripping, protecting and pressing elements, represented by open or closed rings (7,7') or by curved walls (9,9'), form a fantasy shape.

11. The anti-pain device (10) according to any one of the preceding claims, wherein said gripping, protecting and pressing elements (6) are made of the same material as the plate-shaped element (1) or are made of a different material.
